Europäisches Patentamt

European Patent Office

Office européen des brevets

(19)

(11) Veröffentlichungsnummer: **0 186 656**
**B1**

(12) # EUROPÄISCHE PATENTSCHRIFT

(45) Veröffentlichungstag der Patentschrift:
20.09.89

(51) Int. Cl.⁴: **A61B 17/58**

(21) Anmeldenummer: 86100964.5

(22) Anmeldetag: 10.02.84

(60) Veröffentlichungsnummer der früheren Anmeldung
nach Art. 76 EPÜ: 0118778

(54) **Verriegelungsnagel.**

(30) Priorität: 09.03.83 DE 8306675 U

(43) Veröffentlichungstag der Anmeldung:
02.07.86 Patentblatt 86/27

(45) Bekanntmachung des Hinweises auf die Patenterteilung:
20.09.89 Patentblatt 89/38

(84) Benannte Vertragsstaaten:
CH DE FR GB LI

(56) Entgegenhaltungen:
EP-A- 0 008 758
FR-A- 2 342 710
FR-A- 2 387 637
GB-A- 2 114 005

(73) Patentinhaber: Howmedica International, Inc.
Zweigniederlassung Kiel,
Professor-Küntscher-Strasse 1-5, D-2301 Schönkirchen
üb. Kiel(DE)

(72) Erfinder: Richter, Karl Manfred, Haferkamp 14,
D-2304 Wendtorf(DE)
Erfinder: Vecsei, Vilmos, Prof. Dr.,
Marokkanergasse 3/I/39, A-1030 Wien(AT)

(74) Vertreter: Dipl.-Ing. H. Hauck Dipl.-Phys. W. Schmitz
Dipl.-Ing. E. Graalfs Dipl.-Ing. W. Wehnert Dr.-Ing. W.
Döring, Neuer Wall 41, D-2000 Hamburg 36(DE)

## Beschreibung

Die Erfindung bezieht sich auf einen Verriegelungsnagel nach dem Oberbegriff des Patentanspruches.

Ein Verriegelungsnagel dieser Art ist aus FR-A 2 387 637 bekannt geworden. Es ist ein sog. Marknagel, der in den Knochenkanal eingetrieben wird. Über Bildwandler wird die Lage von Querbohrungen im Verriegelungsnagel ermittelt, damit in den Knochen in Übereinstimmung mit den Querbohrungen auch entsprechende Querbohrungen im Knochen geformt werden, über die dann Knochenschrauben in den Knochen und den Verriegelungsnagel eingeschraubt werden. Auf diese Weise kann der Verriegelungsnagel auf beiden Seiten der Fraktur mit dem Knochen fest verbunden werden. Verriegelungsnägel dieser Art dienen insbesondere der Versorgung von Trümmerbrüchen, die mit üblichen Marknägeln (Küntscher-Nagel) nicht ausreichend behandelt werden können. Dies vor allem, weil Trümmerbrüche einen Knochen äußerst rotationslabil machen und die Rotationsstabilität durch einen normalen Marknagel nicht erreicht werden kann.

Der bekannte Verriegelungsnagel weist über eine größte Länge einen achsparallelen Schlitz auf. Ein derartiger Schlitz beeinträchtigt die Rotationsstabilität. Außerdem können mehr oder weniger scharfe Kanten des Schlitzes den Knochen beeinträchtigen. Es besteht daher die Gefahr einer Verletzung oder einer Perforation des Knochens.

Der Erfindung liegt daher die Aufgabe zugrunde, einen Verriegelungsnagel zu schaffen, der sich durch hohe Rotationsstabilität auszeichnet und den Knochen nicht beeinträchtigt.

Diese Aufgabe wird erfindungsgemäß gelöst durch die Merkmale des Kennzeichnungsteils des Patentanspruches.

Das Querschnittsprofil des erfindungsgemäßen Verriegelungsnagels weist über den Umfang abwechselnd in gleichmäßigen Umfangsabständen Erhebungen und Vertiefungen auf. Die Übergänge zwischen Erhebung und Vertiefung sind stetig und möglichst weich, um die Verletzungsgefahr zu vermindern. Die radial am weitesten außen und radial am weitesten innen liegenden Punkte der Erhebungen und Vertiefungen liegen auf einer Kreisbahn konzentrisch zur Achse. Die Erhebungen sind gerundete Zahnspitzen, während die Vertiefungen dazwischen liegende Kannelierungen bilden mit weichem Übergang zu den Erhebungen.

Das ringförmig geschlossene Querschnittsprofil des erfindungsgemäßen Nagels weist ein hohes Widerstandsmoment auf und ergibt mithin eine hohe Rotationsstabilität. Die von der Kreisform abweichende Außenkontur des Nagels ermöglicht einen mehr oder weniger ausgeprägten Eingriff im Knochenkanal, um eine Relativdrehung zwischen Knochen und Nagel zu vermeiden. Gleichzeitig ist der Dreheingriff jedoch derart, daß eine Verletzung oder gar Perforation des Knochens ausgeschlossen wird, und zwar durch die gerundeten Übergänge. Es wäre zwar denkbar, die Außenkontur mit mehr oder weniger scharfen schneidenartigen Kanten zu versehen, um einen besonders ausgeprägten Dreheingriff zu erhalten. Eine derartige Kontur würde jedoch, wie schon ausgeführt, zu einer möglichen Verletzung des Knochens führen.

Derartige Nägel, die einen Durchmesser von 9 bis 18 mm aufweisen können, werden häufig mit Hilfe eines sogenannten Führungsspießes eingetrieben, der zuvor in den Knochen eingetrieben wurde. Zu diesem Zweck muß der Nagel einen in Längsrichtung durchgehenden Kanal für den Führungsspieß aufweisen. Der Innenkanal ist daher im Querschnitt kreisförmig.

Das Material des erfindungsgemäßen Nagels besteht naturgemäß aus körperverträglichem, nicht korrodierendem Material. Es ist gleichzeitig geeignet, im Strangpreßverfahren hergestellt zu werden. Das Strangpreßverfahren verhindert die Entstehung von Nähten oder Graten am Umfang des Nagels, welche äußerst unerwünscht sind.

Ausführungsbeispiele der Erfindung werden nachfolgend anhand von Zeichnungen erläutert.

Fig. 1 zeigt einen Schnitt durch einen Verriegelungsnagel, der in einem Oberschenkelknochen implantiert ist.

Fig. 2 zeigt einen Schnitt durch den Verriegelungsnagel nach Fig. 1.

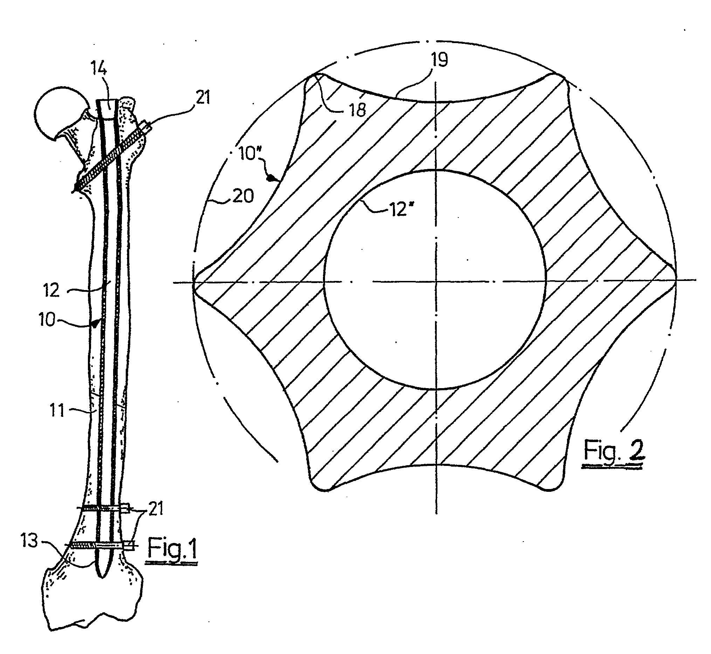

In Fig. 1 ist ein Verriegelungsnagel 10 vom Trochanter major in einen Oberschenkelknochen 11 eingetrieben. Der Verriegelungsnagel 10 ist ein Hohlprofil mit einem durchgehenden inneren Kanal 12, der bis zur verjüngten Spitze 13 des Nagels 10 reicht. Auf diese Weise kann bei Verwendung eines Führungsspießes der Nagel 10 den Führungsspieß entlang eingetrieben werden (wie dies in Verbindung mit der Nagelung nach Küntscher allgemein bekannt ist). Am Einschlagende weist der Nagel 10 eine konische Erweiterung 14 auf mit einem Innengewinde, mit dem ein Einschlagwerkzeug in Eingriff gebracht werden kann.

Die Nagelungsmethode erfordert, daß nach dem Eintreiben des Verriegelungsnagels 10 die Lage von Paaren von Querbohrungen mit Hilfe eines Bildwandlers ermittelt wird, um in der gemeinsamen Achse eines Bohrungspaares ein Loch in die Cortikalis zu bohren zwecks Einschraubens von Querschrauben 15. Bei der Darstellung nach Fig. 1 sind beidseits der Trümmerfraktur jeweils Querknochenschrauben 15 eingeschraubt. Die oberste ist dabei schräg eingesetzt, entsprechend den hierfür vorgesehenen Bohrungen im Nagel 10.

In Fig. 2 ist der Querschnitt eines Verriegelungsnagels 10" dargestellt, dessen Außenkontur einem Malteserkreuz gleicht mit sechs in Umfangsrichtung gleichmäßig beabstandeten Zahnspitzen 18, die kreisbogenförmig gerundet sind. Zwischen den Zahnspitzen findet sich eine entsprechende Kannelierung 19, die mit weichem Übergang in die Spitzen 18 übergeht. Die Zahnspitzen 18 liegen auf einem Kreisbogen 20 konzentrisch zur Achse des Nagels 10". Der Innenkanal 12" ist im Querschnitt kreisförmig.

**Patentansprüche**

1. Verriegelungsnagel zur Versorgung von Knochenfrakturen bestehend aus einem länglichen Körper mit kreisförmigen Innenkanal (12), wobei der Verriegelungsnagel am vorderen Ende (13) verjüngt ist und am Einschlagende eine Erweiterung (14) aufweist zur Aufnahme eines Einschlagwerkzeugs, und mindestens ein Paar in Längsrichtung des Nagels beabstandete Querbohrungen aufweist zur Aufnahme von beidseits einer Fraktur angeordneten Knochenschrauben, dadurch gekennzeichnet, daß das Querschnittsprofil des Verriegelungsnagels über seinen Umfang ringförmig geschlossen ist und der Radius der Außenfläche über den Umfang sich verändert derart, daß die radial am weitesten außen und am weitesten innen liegenden Punkte der Erhebungen (18) und Vertiefungen (19) auf einem Kreisbogen (20) konzentrisch zur Achse liegen, wobei die Übergänge zwischen den in gleichmäßigen Umfangsabständen liegenden Erhebungen (18) und Vertiefungen (19) stetig und weich sind und die Erhebungen (18) als kreisbogenförmig gerundete Zahnspitzen ausgebildet sind und zwischen sich Kannelierungen bilden, die mit weichem Übergang in die Erhebungen (18) übergehen.

**Claims**

1. An interlocking nail for the treatment of bone fractures comprising an elongated body with a circular inner passage (12), wherein the interlocking nail is tapered at the front end (13) and at the end of driving-in is provided with an enlargement (14) for the accommodation of a tool for driving-in and has at least one pair of transverse bores being spaced in longitudinal direction of the nail for the accommodation of bone screws being positioned at both sides of a fracture, characterized in that the cross-sectional profile of the interlocking nail over the circumference thereof is closed in the shape of a ring and the radius of the outer surface changes in such a manner that the sites of the elevations (18) and deepenings (19) disposed radially farthest outward and farthest inward lie on a circular arc (20) concentric with respect to the axis wherein the transitions between the elevations (18) and deepenings (19) being uniformly spaced around the circumference are continuous and smooth and the elevations (18) are defined as tips of teeth which are rounded in the shape of a circular arc and form flutes therebetween which comprise smooth transitions towards the elevations (18).

**Revendications**

1. Clou d'ancrage destiné au traitement des fractures osseuses, consistant en un corps longitudinal à canal intérieur circulaire (12), le clou d'ancrage étant rétréci à l'extrémité avant (13) et présentant un élargissement (14) à l'extrémité de tête de frappe pour recevoir un outil d'enfoncement, et présentant au moins une paire de perçages transversaux distants l'un de l'autre dans la direction longitudinale du clou pour recevoir des vis d'ostéoplastie disposées des deux côtés d'une fracture, caractérisé en ce que le profil transversal du clou d'ancrage est en anneau fermé sur sa périphérie et le rayon de la surface extérieure varie sur la périphérie d'une manière telle que les points situés radialement le plus loin vers l'extérieur et le plus loin vers l'intérieur des saillies (18) et des évidements (19) sont disposés sur un arc de cercle (20) concentriquement à l'axe, les raccords entre les saillies (18) et les évidements (19) situés à des distances périphériques égales étant continus et adoucis, et les saillies (18) présentent une structure en pointes de dents arrondies en forme d'arc de cercle et constituent entre elles des cannelures qui se raccordent par un raccord adouci avec les saillies (18)

14

21

12

10

11

21

13

Fig.1

19

18

10"

20

12"

Fig. 2